(19) Europäisches Patentamt
European Patent Office
Office européen des brevets

(11) Veröffentlichungsnummer: **0 033 501**
**A2**

(12) # EUROPÄISCHE PATENTANMELDUNG

(21) Anmeldenummer: **81100528.9**

(22) Anmeldetag: **24.01.81**

(51) Int. Cl.³: **C 07 D 233/60**
A 01 N 43/50

(30) Priorität: **04.02.80 DE 3003933**

(43) Veröffentlichungstag der Anmeldung: **12.08.81 Patentblatt 81/32**

(84) Benannte Vertragsstaaten: **AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Pommer, Ernst-Heinrich, Dr.**
**Berliner Platz 7**
**D-6703 Limburgerhof(DE)**

(72) Erfinder: **Scharwaechter, Peter, Dr.**
**An der Duene 9**
**D-2082 Morrege(DE)**

(72) Erfinder: **Gutsche, Klaus, Dr.**
**Ehmschenkamp 5**
**D-2084 Rellingen(DE)**

(54) **Beta-Imidazolylalkohole, Verfahren zu ihrer Herstellung, diese enthaltende Fungizide und Verfahren zur Bekämpfung von Pilzen mit ihnen.**

(57) β-Imidazolylalkohol der Formel

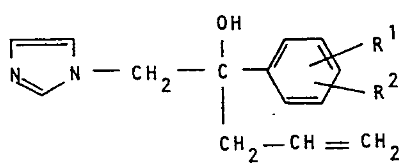

in der $R^1$ Wasserstoff, Halogen oder Methyl und $R^2$ Wasserstoff oder Halogen bedeuten und dessen Salze und Metallkomplexverbindungen sowie diese enthaltende Fungizide, Verfahren zu ihrer Herstellung und ihre Anwendung als Fungizide.

EP 0 033 501 A2

Croydon Printing Company Ltd.

# ß-Imidazolylalkohole, Verfahren zu ihrer Herstellung, diese enthaltende Fungizide und Verfahren zur Bekämpfung von Pilzen mit ihnen

Die vorliegende Erfindung betrifft neue, wertvolle ß-Imidazolylalkohole, insbesondere neue 1-(Imidazol-1-yl)-2-phenyl-pent-4-en-2-ole, Verfahren zu ihrer Herstellung, diese enthaltende Fungizide und ihre Verwendung zur Bekämpfung von Pilzen.

Es ist bekannt, daß das 1-[2-(2,4-Dichlorphenyl)-2-(2-propenyloxy)-äthyl]-imidazol eine fungizide Wirksamkeit hat (DE-OS 20 63 857). Seine Wirkung ist insbesondere gegenüber Mehltaupilzen und Rostpilzen unzureichend. Aus diesem Grunde ist es zur Bekämpfung von Pilzen wenig geeignet.

Es wurde gefunden, daß ß-Imidazolylalkohole der Formel

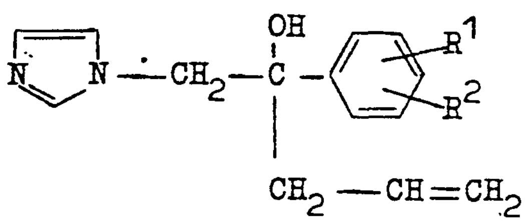

in der $R^1$ Wasserstoff, Halogen (F, Cl, Br) oder Methyl und $R^2$ Wasserstoff oder Halogen (F, Cl, Br) bedeuten, und ihre Salze und Metallkomplexverbindungen gut wirksam gegen Schadpilze, insbesondere aus der Klasse der Ascomyceten und Basidiomyceten, sind. Salze sind insbesondere die Säureadditionssalze, z.B. Hydrochloride, Hydrobromide, Sulfate, Oxalate, Dodecylbenzolsulfonate oder Nitrate.

Die ß-Imidazolylalkohole und ihre Salze können hergestellt werden, indem man einen Alkohol der Formel

Sws/BL

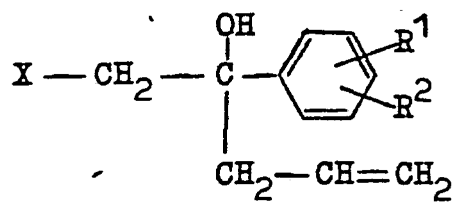

in welcher $R^1$ und $R^2$ die oben angegebenen Bedeutungen haben und X für Halogen, vorzugsweise Chlor oder Brom, steht, mit Imidazol, gegebenenfalls in Gegenwart eines säurebindenden Mittels, oder mit seinem Alkalisalz, wie es beispielsweise aus Imidazol durch Behandlung mit Natriummethylat in einem geeigneten Lösungsmittel erhalten wird, umsetzt und anschließend gegebenenfalls den erhaltenen ß-Imidazolylalkohol mit einer Säure in sein Salz überführt.

Die Umsetzung des Alkohols mit Imidazol oder dessen Alkalisalzen kann sowohl in Gegenwart als auch in Abwesenheit von Verdünnungsmitteln ausgeführt werden. Als Verdünnungsmittel werden vorwiegend organische Lösungsmittel, beispielsweise Dimethylformamid, Hexamethylphosphortriamid, Acetonitril oder Benzol verwendet.

Die Umsetzung wird, sofern nicht ein Alkalisalz des Imidazols eingesetzt wird, vorzugsweise in Gegenwart eines Überschusses, mindestens jedoch in Gegenwart von etwa der stöchiometrischen Menge eines säurebindenden Mittels durchgeführt. Als säurebindendes Mittel eignet sich vorzugsweise ein Überschuß des eingesetzten Imidazols. Weiterhin eignen sich alle üblichen Säurebindungsmittel, z.B. Hydroxide, Carbonate und Alkoholate von Alkali- und Erdalkalimetallen sowie organische Basen z.B. tertiäre Amine.

Die Reaktionstemperaturen können in einem größeren Bereich variiert werden. Im allgemeinen arbeitet man bei 0 bis

150°C, vorzugsweise bei Temperaturen zwischen 30 und 120°C.

Die Herstellung des Alkohols kann beispielsweise nach den Methoden zur Darstellung tertiärer Alkohole aus den entsprechenden Ketonen oder Carbonsäurederivaten und Grignard-Verbindungen durchgeführt werden (Lit.: Houben-Weyl, Methoden der organischen Chemie, 13/2a, 46-527 (1973)).

Die Alkohole werden beispielsweise dadurch erhalten, daß man ein Keton der Formel

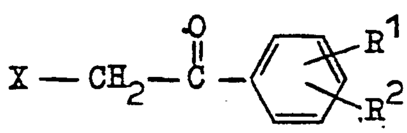

in welcher $R^1$, $R^2$ und X die oben angegebene Bedeutung haben, mit einer Grignard-Verbindung der Formel

$$Y\text{-}Mg\text{-}CH_2\text{-}CH{=}CH_2$$

in welcher Y für Halogen, vorzugsweise Chlor oder Brom, steht, in einem für Grignard-Reaktionen üblichen Lösungsmittel, vorzugsweise Diäthyläther oder Tetrahydrofuran, umsetzt.

Die Alkohole werden im allgemeinen als Rohprodukte aus der Reaktionsmischung abgetrennt und mit einem Imidazol, wie oben beschrieben, umgesetzt. In besonderen Fällen können die Alkohole jedoch nach ihrer Herstellung abgetrennt und danach mit einem Imidazol umgesetzt werden, was zur Erzielung höherer Ausbeuten von Vorteil sein kann.

Die ß-Imidazolylalkohole kommen als optisch aktive isomere d- und l-Form und als ihre Mischung (Racemate) vor.

Unter der Bezeichnung ß-Imidazolylalkohole werden die zwei Isomere und ihre Mischung verstanden.

Beispiel 1

4,01 g Magnesiumspäne werden in 200 ml Diäthyläther mit 18,15 g Allylbromid bei Siedetemperatur zur Reaktion gebracht. Zu dieser Lösung läßt man anschließend 15,4 g ω-Chloracetophenon, gelöst in 150 ml Diäthyläther, zutropfen und rührt bei dieser Temperatur. Anschließend wird mit wäßriger Ammoniumchloridlösung versetzt, die organische Phase abgetrennt und über Natriumsulfat getrocknet. Nach Einengen im Vakuum wird der Rückstand in 75 ml Dimethylformamid gelöst und umgesetzt mit einer Lösung von Natriumimidazol in 75 ml Dimethylformamid, hergestellt aus 2,3 g Natrium, umgesetzt mit 50 ml Methanol und 6,81 g Imidazol und gelöst im Dimethylformamid, und die Mischung über Nacht bei Raumtemperatur gerührt. Das Lösungsmittel wird im Vakuum abdestilliert, der Rückstand in Chloroform gelöst, mit Wasser gewaschen, über Natriumsulfat getrocknet und im Vakuum eingeengt. Nach Lösen in Diäthyläther und Umkristallisieren aus Benzol werden 15,98 g (70 % der Theorie) kristallines 1-(Imidazol-1-yl)-2-phenyl-pent-4-en-2-ol mit dem Schmelzpunkt 131°C erhalten (Wirkstoff Nr. 1).

Beispiel 2

4,01 g Magnesiumspäne werden in 200 ml Diäthyläther mit 18,15 g Allylbromid bei Siedetemperatur zur Reaktion gebracht. Zu dieser Lösung läßt man schließend 15,46 g ω-Chloracetophenon, gelöst in 150 ml Diäthyläther, zutropfen. Man zersetzt anschließend mit wäßriger Ammoniumchloridlösung, trennt die organische Phase ab, wäscht sie neutral und trocknet sie über Natriumsulfat. Nach Einengen der Phase im Vakuum wird der Rückstand in eine Schmelze von

34,04 g Imidazol gegeben und unter Rühren bei 120°C zur Reaktion gebracht. Man kühlt danach ab und versetzt mit Wasser und Chloroform, wäscht die Chloroformlösung neutral, trocknet sie über Natriumsulfat und versetzt sie bei 50°C mit einer Diisopropyläther-HCl-Lösung. Nach Umkristallisation der abgetrennten Festsubstanz aus Aceton erhält man 17,21 g (65 % der Theorie) kristallines 1-(Imidazol-1-yl)-2-phenyl-pent-4-en-2-ol-hydrochlorid mit dem Schmelzpunkt 141°C (Wirkstoff Nr. 2).

Entsprechend wurden die folgenden Verbindungen hergestellt:

3. 1-(Imidazol-1-yl)-2-(4-chlorphenyl)-pent-4-en-2-ol-hydrochlorid mit dem Fp.: 178°C.

4. 1-(Imidazol-1-yl)-2-(4-chlorphenyl)-pent-4-en-2-ol-nitrat mit dem Fp.: 155°C.

5. 1-(Imidazol-1-yl)-2-(2,4-dichlorphenyl)-pent-4-en-2-ol mit dem Fp.: 117°C.

6. 1-(Imidazol-1-yl)-2-(2,4-dichlorphenyl)-pent-4-en-2-ol-hydrochlorid mit dem Fp.: 173°C.

7. 1-(Imidazol-1-yl)-2-(2,4-dichlorphenyl)-pent-4-en-2-ol-nitrat mit dem Fp.: 145°C.

8. 1-(Imidazol-2-yl)-2-(3,4-dichlorphenyl)-pent-4-en-2-ol mit dem Fp.: 157°C.

9. 1-(Imidazol-1-yl)-2-(3,4-dichlorphenyl)-pent-4-en-2-ol-hydrochlorid mit dem Fp.: 172°C.

10. 1-(Imidazol-1-yl)-2-(4-bromphenyl)-pent-4-en-2-ol mit dem Fp.: 172°C.

11. 1-(Imidazol-1-yl)-2-(4-bromphenyl)-pent-4-en-2-ol-hydrochlorid mit dem Fp.: 182°C.

12. 1-(Imidazol-1-yl)-2-(4-fluorphenyl)-pent-4-en-2-ol mit dem Fp.: 167°C.

13. 1-(Imidazol-1-yl)-2-(4-fluorphenyl)-pent-4-en-2-ol-hydrochlorid mit dem Fp.: 169°C.

14. 1-(Imidazol-1-yl)-2-(2-brom-4-chlor-phenyl)-pent-4-en-2-ol-hydrochlorid mit dem Fp.: 156°C.

15. 1-(Imidazol-1-yl)-2-(2-brom-4-chlor-phenyl)-pent-4-en-2-ol-nitrat mit dem Fp.: 138°C (Zersetzung).

16. 1-(Imidazol-1-yl)-2-(4-methyl-phenyl)-pent-4-en-2-ol mit dem Fp.: 155°C.

17. 1-(Imidazol-1-yl)-2-(4-methyl-phenyl)-pent-4-en-2-ol-hydrochlorid mit dem Fp.: 174°C.

Die Metallkomplexverbindungen der neuen ß-Imidazolylalkohole und ihre Salze entsprechen der Formel

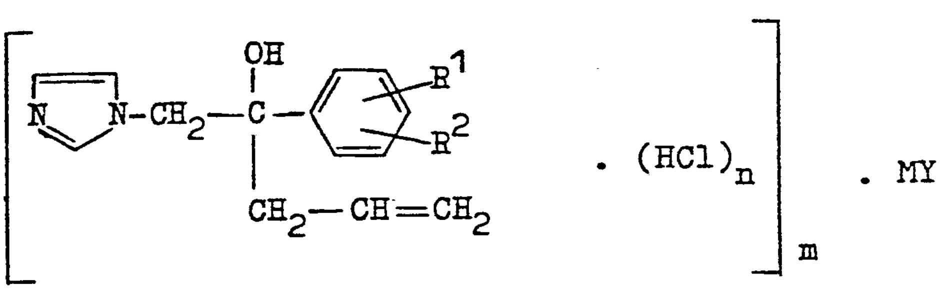

in welcher
$R^1$ und $R^2$ die oben genannten Bedeutungen haben und
M für Kupfer oder Zink steht,
Y für Sulfat oder 2 Chlorid steht,
n für 0 oder 1 steht und
m für 2 oder 4 steht.

Die Metallkomplexverbindungen erhält man, indem man einen ß-Imidazolylalkohol der Formel

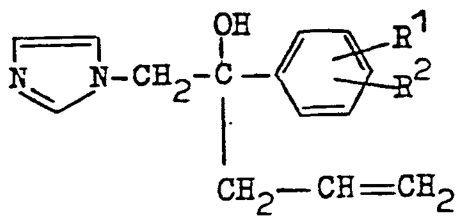 $\cdot\ (HCl)_n$

in welcher
$R^1$, $R^2$ und n die oben angegebene Bedeutung haben, mit einem Metallsalz der Formel

$$MY \cdot k\ H_2O$$

in welcher k für eine beliebige ganze Zahl von 0 bis 12 steht und M und Y die oben angegebene Bedeutung haben, in Gegenwart eines Lösungsmittels umsetzt.

Verwendet man beispielsweise 1-(Imidazol-1-yl)-2-(2,4-dichlorphenyl)-pent-4-en-2-ol und Kupfer-II-chlorid als Ausgangsstoffe, so kann der Reaktionsablauf durch das folgende Formelschema wiedergegeben werden:

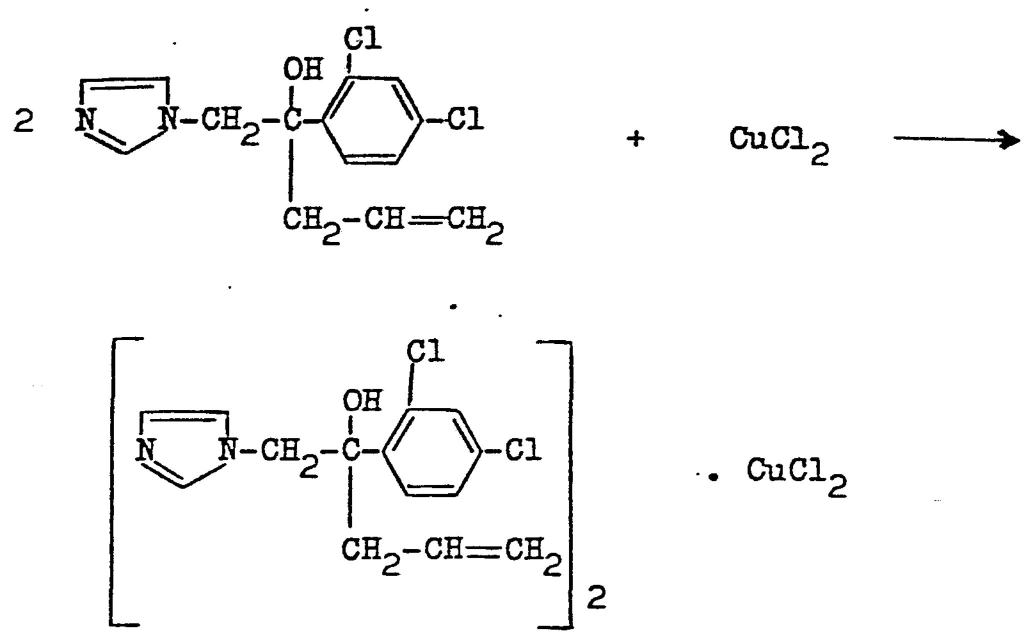


Für die Herstellung der Metallkomplexverbindungen kommen als Verdünnungsmittel Wasser; Alkohole, wie Methanol und Äthanol; Ketone, z.B. Aceton; oder Äther, z.B. Diäthyläther oder Dioxan, in Frage.

Die Reaktionstemperaturen können in einem größeren Bereich variiert werden. Im allgemeinen arbeitet man zwischen 0 und 80°C, vorzugsweise zwischen 15 und 50°C.

Beispiel 18

6 g 1-(Imidazol-1-yl)-2-(2,4-dichlorphenyl)-pent-4-en-2-ol und 1,71 g Kupfer-(II)-chlorid-dihydrat werden in 200 ml Methanol gelöst und 30 Minuten bei 50°C gerührt. Nach der Filtration der Reaktionsmitschung engt man sie im Vakuum ein und behandelt sie mit Diisopropyläther. Man erhält als Fällung 7,04 g (98 % der Theorie) an grünem Bis-[1-

-(imidazol-1-yl)-2-(2,4-dichlorphenyl)-pent-4-en-2-ol]-kupfer-(II)-chlorid mit dem Schmelzpunkt 104°C (Wirkstoff Nr. 18).

Beispiel 19

16,1 g 1-(Imidazol-1-yl)-2-(2,4-dichlorphenyl)-pent-4-en-2-ol-hydrochlorid und 4,11 g Kupfer-(II)-chlorid-dihydrat werden in 400 ml Methanol gelöst und 30 Minuten bei 50°C gerührt. Nach dem Abkühlen wird die Reaktionsmischung mit Diisopropyläther versetzt, wobei sich 16 g (99 % der Theorie) gelbkristallines Bis-[1-(imidazol-1-yl)-2-(2,4-dichlorphenyl)-pent-4-en-2-ol-hydrochlorid]-kupfer-(II)-chlorid mit dem Schmelzpunkt 163°C abscheiden (Wirkstoff Nr. 19).

Beispiel 20

6 g 1-(Imidazol-1-yl)-2-(2,4-dichlorphenyl)-pent-4-en-2-ol und 1,37 g Zinkchlorid werden in 100 ml Methanol in der Wärme gelöst, filtriert, im Vakuum eingeengt, auf 300 ml Eiswasser gegeben und das ausgefallene Salz filtriert. Man erhält 7,18 g (98 % der Theorie) weißkristallines Bis-[1-(imidazol-1-yl)-2-(2,4-dichlorphenyl)-pent-4-en-2-ol]-zinkchlorid mit dem Schmelzpunkt 92°C (Wirkstoff Nr. 20).

Entsprechend wurden folgende Verbindungen hergestellt:

21. Bis-[-(imidazol-1-yl)-2-phenyl-pent-4-en-2-ol]-kupfer-(II)-chlorid mit dem Fp.: 92°C.

22. Bis-[1-(imidazol-1-yl)-2-phenyl-pent-4-en-2-ol-hydrochlorid]-kupfer-(II)-chlorid mit dem Fp.: 153°C.

23. Bis-[1-(imidazol-1-yl)-2-(4-fluorphenyl)-pent-4-en-2-ol]-kupfer-(II)-chlorid mit dem Fp.: 99°C.

24. Bis-[1-(imidazol-1-yl)-2-(4-fluorphenyl)-pent-4-en-2-ol-hydrochlorid]-kupfer-(II)-chlorid mit dem Fp.: 159°C.

25. Bis-[1-(imidazol-1-yl)-2-(4-bromphenyl)-pent-4-en-2-ol]-kupfer-(II)-chlorid mit dem Fp.: 177°C.

26. Bis-[1-(imidazol-1-yl)-2-(4-chlorphenyl)-pent-4-en-2-ol-hydrochlorid]-kupfer-(II)-chlorid mit dem Fp.: 165°C.

27. Bis-[1-(imidazol-1-yl)-2-(3,4-dichlorphenyl)-pent-4-en-2-ol]-kupfer-(II)-chlorid mit dem Fp.: 101°C.

28. Bis-[1-(imidazol-1-yl)-2-(3,4-dichlorphenyl)-pent-4-en-2-ol-hydrochlorid]-kupfer-(II)-chlorid mit dem Fp.: 163°C.

29. Bis-[1-(imidazol-1-yl)-2-(4-brom-2-chlorphenyl)-pent-4-en-2-ol-hydrochlorid]-kupfer-(II)-chlorid mit dem Fp.: 139°C.

30. Tetra-[1-(imidazol-1-yl)-2-(2,4-dichlorphenyl)-pent-4-en-2-ol]-kupfer-(II)-sulfat mit dem Fp.: 156°C.

31. Tetra-[1-(imidazol-1-yl)-2-(2,4-dichlorphenyl)-pent-4-en-2-ol-hydrochlorid]-kupfer-(II)-sulfat mit dem Fp.: 87°C.

32. Bis-[1-(imidazol-1-yl)-2-(2,4-dichlorphenyl)-pent-4-en-2-ol-hydrochlorid]-zinkchlorid mit dem Fp.: 153°C.

Die neuen Verbindungen und ihre Salze und Metallkomplexverbindungen zeigen eine starke fungitoxische Wirksamkeit gegen phytophathogene Pilze, insbesondere aus den Klassen der Ascomyceten, Basidiomyceten, Phycomyceten und der Sammelgruppe der Fungi imperfecti. Folgende Pilze sind beispielsweise zu nennen: Erysiphe graminis an Weizen, Erysiphe cichoriacearum an Gurken, Uncinula necator an Reben, Podosphaera leucotricha an Äpfeln, Septoria glycines an Sojabohnen, Cercosporella herpotrichoides an Weizen, Cercospora personata an Erdnüssen, Venturia inaequalis an Äpfeln, Puccinia-Arten an Getreide, Uromyces phaseoli an Bohnen, Plasmopara viticola an Reben, Phytophthora infestans an Tomaten und Kartoffeln, Botrytis cinerea an Reben, Erdbeeren, Zierpflanzen und Solanaceen, Septoria nodorum an Weizen, Mycosphaerella musicola an Bananen, Piricularia oryzae an Reis, Diplodia natalensis an Citrus, Alternaria solani an Tomaten und Kartoffeln, Hemileia vastatrix an Kaffee.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung oder wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel

als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine oder Amide (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen - Fettalkohole - Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,01 und 3 kg Wirkstoff oder mehr je ha, vorzugsweise zwischen 0,01 und 1 kg/ha.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung des Beispiels 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Beispiels 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

III. 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

IV. 20 Gewichtsteile der Verbindung des Beispiels 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

V. 20 Gewichtsteile der Verbindung des Beispiels 3 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile der Verbindung des Beispiels 1 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 3 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,0 Gew.-% Wirkstoff enthält.

IX. 20 Teile der Verbindung des Beispiels 1 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie Ferridimethyldithiocarbamat, Zinkdimethyldithiocarbamat, Manganethylenbisdithiocarbamat, Mangan-Zink-ethylendiamin-bis-dithiocarbamat oder Zinkethylenbisdithiocarbamat,
Tetramethylthiuramidsulfide,
Ammoniak-Komplex von Zink-(N,N-ethylen-bis-dithiocarbamat) und N,N'-Polyethylen-bis-(thiocarbamoyl)-disulfid,
Zink-(N,N'-propylen-bis-dithiocarbamt),
Ammoniak-Komplex von Zink-(N,N'-propylen-bis-dithiocarbamat) und N,N'-Propylen-bis(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
heterocyclische Substanzen, wie
N-Trichlormethylthio-tetrahydrophthalimid,
N-(1,1,2,2-Tetrachlorethylthio)-tetrahydrophthalimid,
N-Trichlormethylthio-phthalimid,

2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-pyridinmethanol,
1,2-Bis-(3-ethoxycarbonyl-2-thioureido)-benzol,
1,2-Bis-(3-methoxycarbonyl-2-thioureido)-benzol

und weitere Substanzen wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,

2-Methyl-benzoesäure-anilid,
2-Jod-Benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Die folgenden Ausführungen zeigen die biologische Wirkung der neuen Substanzen. Als Vergleichssubstanz diente das aus der DE-OS 20 63 857 bekannte 1-Imidazolyl-2-(2,4-dichlorphenyl)-2-(2-propenyloxy)-ethan (Wirkstoff A) sowie das bekannte Zink-ethylen-1,2-bisdithiocarbamat (Wirkstoff B).

<u>Wirksamkeit gegen Weizenmehltau</u>

Blätter von in Töpfen gewachsenen Weizenkeimlingen der Sorte "Jubilar" werden mit wäßrigen Emulsionen aus 80 % (Gew.%) Wirkstoff und 20 % Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach

10 Tagen wird das Ausmaß der Mehltauentwicklung ermittelt.

Das Ergebnis des Versuches zeigt, daß die Wirkstoffe Nr. 2, 3, 6, 9, 11, 13, 18, 20, 21, 22, 23, 24, 25, 27, 28, 29, 30, 31, 32 eine bessere fungizide Wirkung als das Vergleichsmittel A haben.

Wirksamkeit gegen Gurkenmehltau

Blätter von in Töpfen gewachsenen Gurkenkeimlingen werden mit wäßrigen Emulsionen aus 80 % Wirkstoff und 20 % Emulgiermittel besprüht und nach dem Antrocknen des Spritzbelages mit Oidien (Sporen) des Gurkenmehltaus (Erysiphe cichoriacearum) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltaupilzentwicklung ermittelt.

Das Ergebnis des Versuches zeigt, daß die Wirkstoffe Nr. 2, 3, 6, 9, 11, 13, 18, 19, 20, 21, 22, 23, 24, 25, 26, 27, 28, 29, 30, 31, 32 eine gute fungizide Wirkung haben.

Wirksamkeit gegen Weizenbraunrost

Blätter von in Töpfen gewachsenen Weizenpflanzen der Sorte "Jubilar" werden mit Sporen des Weizenbraunrostes (Puccinia recondita) künstlich infiziert und 48 Stunden lang bei 20 bis 25°C in einer wasserdampfgesättigten Kammer aufgestellt. Danach werden die Pflanzen mit wäßrigen Spritzbrühen, die in dem Wasser gelöst oder emulgiert eine Mischung aus 80 % des zu prüfenden Wirkstoffes und 20 % Natriumligninsulfonat enthalten, besprüht und im Gewächshaus bei Temperaturen zwischen 20 und 22°C und bei 75 bis 80 % relativer Luftfeuchtigkeit aufgestellt. Nach 10 Tagen wird das Ausmaß der Rostpilzentwicklung beurteilt.

Das Ergebnis des Versuches zeigt, daß die Wirkstoffe Nr. 2, 6, 9, 11, 13, 18, 19, 20, 25, 27, 30, 31, 32 eine gute fungizide Wirkung haben.

Wirksamkeit gegen Phytophthora infestans an Tomaten

Blätter von Topfpflanzen der Sorte "Große Fleischtomate" werden mit wäßrigen Suspensionen, die 0,025 % (Gew.%) Wirkstoff enthalten, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß die Wirkstoffe Nr. 18, 20, 21, 22, 23, 24, 25, 27, 29, 30, 31, 32 eine bessere fungizide Wirkung als das Vergleichsmittel B haben.

Botrytis cinerea an Paprika

Paprikasämlinge der Sorte "Neusiedler Ideal Elite" werden, nachdem sich 4 bis 5 Blätter gut entwickelt haben, mit 0,1 % (Gew.%) wäßrigen Suspensionen, die 80 % Wirkstoff und 20 % Natriumligninsulfonat in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt,

um optimale Bedingungen für die Pilzentwicklung zu erhalten. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuchs zeigt, daß die neuen Wirkstoffe 18, 21, 25, 30, 31, 32 eine gute fungizide Wirksamkeit haben.

## Patentansprüche

1. ß-Imidazolylalkohol der Formel

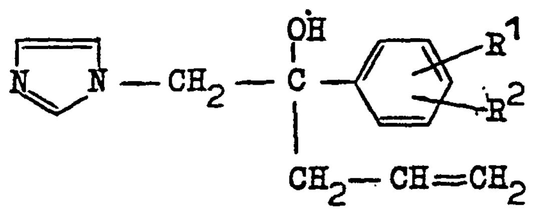

in der $R^1$ Wasserstoff, Halogen oder Methyl und $R^2$ Wasserstoff oder Halogen bedeuten und dessen Salze und Metallkomplexverbindungen.

2. Fungizid, enthaltend einen ß-Imidazolylalkohol gemäß Anspruch 1.

3. Verfahren zur Bekämpfung von Pilzen, dadurch gekennzeichnet, daß man die Pilze oder die vor Pilzbefall zu schützenden Gegenstände behandelt mit einem ß-Imidazolylalkohol gemäß Anspruch 1.

4. Verfahren zur Herstellung eines ß-Imidazolylalkohols gemäß Anspruch 1, dadurch gekennzeichnet, daß man ein Imidazol umsetzt mit einem Alkohol der Formel

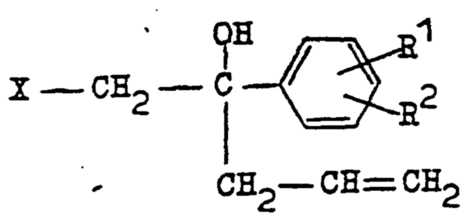

in der X Halogen bedeutet und $R^1$ und $R^2$ die im Anspruch 1 genannten Bedeutungen haben.

5. ß-Imidazolylalkohol gemäß Anspruch 1, ausgewählt aus der Gruppe, bestehend aus
1-(Imidazol-1-yl)-2-phenyl-pent-4-en-2-ol,
1-(Imidazol-1-yl)-2-(4-chlorphenyl)-pent-4-en-2-ol,
1-(Imidazol-1-yl)-2-(2,4-dichlorphenyl)-pent-4-en-2-ol,
1-(Imidazol-1-yl)-2-(3,4-dichlorphenyl)-pent-4-en-2-ol,
1-(Imidazol-1-yl)-2-(4-bromphenyl)-pent-4-en-2-ol und
1-(Imidazol-1-yl)-2-(4-fluorphenyl)-pent-4-en-2-ol.

6. ß-Imidazolylalkohol-Metallkomplexverbindung gemäß Anspruch 1, ausgewählt aus der Gruppe, bestehend aus
Bis-[1-(imidazol-1-yl)-2-(2,4-dichlorphenyl)-pent-4-en-2-ol]-kupfer-(II)-chlorid,
Bis-[1-(imidazol-1-yl)-2-(2,4-dichlorphenyl)-pent-4-en-2-ol]-kupfer-(II)-sulfat,
Bis-[1-(imidazol-1-yl)-2-(3,4-dichlorphenyl)-pent-4-en-2-ol]-kupfer-(II)-chlorid,
Tetra-[1-(imidazol-1-yl)-2-(2,4-dichlorphenyl)-pent-4-en-2-ol]-kupfer-(II)-sulfat und
Bis-[1-(imidazol-1-yl)-2-(2,4-dichlorphenyl)-pent-4-en-2-ol]-zinkchlorid.

Patentansprüche (für Österreich)

1. Fungizid, enthaltend einen ß-Imidazolylalkohol der Formel

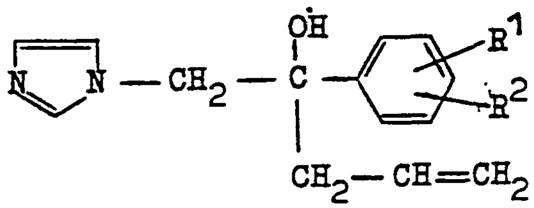

in der $R^1$ Wasserstoff, Halogen oder Methyl und $R^2$ Wasserstoff oder Halogen bedeuten oder dessen Salze oder Metallkomplexverbindungen.

2. Fungizid, enthaltend einen festen oder flüssigen Trägerstoff und einen ß-Imidazolylalkohol wie in Anspruch 1 definiert.

3. Verfahren zur Bekämpfung von Pilzen, dadurch gekennzeichnet, daß man die Pilze oder die vor Pilzbefall zu schützenden Gegenstände behandelt mit einem ß-Imidazolylalkohol wie in Anspruch 1 definiert.

4. Verfahren zur Herstellung eines ß-Imidazolylalkohols wie in Anspruch 1 definiert, dadurch gekennzeichnet, daß man ein Imidazol umsetzt mit einem Alkohol der Formel

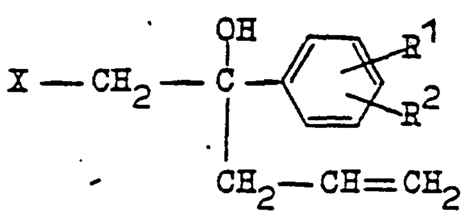

in der X Halogen bedeutet und $R^1$ und $R^2$ die im Anspruch 1 genannten Bedeutungen haben.

5. Fungizid gemäß Anspruch 1 enthaltend einen ß-Imidazolylalkohol ausgewählt aus der Gruppe, bestehend aus
1-(Imidazol-1-yl)-2-phenyl-pent-4-en-2-ol,
1-(Imidazol-1-yl)-2-(4-chlorphenyl)-pent-4-en-2-ol,
1-(Imidazol-1-yl)-2-(2,4-dichlorphenyl)-pent-4-en--2-ol,
1-(Imidazol-1-yl)-2-(3,4-dichlorphenyl)-pent-4-en--2-ol,
1-(Imidazol-1-yl)-2-(4-bromphenyl)-pent-4-en-2-ol und
1-(Imidazol-1-yl)-2-(4-fluorphenyl)-pent-4-en-2-ol.